(19) Europäisches Patentamt
European Patent Office
Office européen des brevets

(11) EP 3 136 269 A1

# (12) EUROPEAN PATENT APPLICATION

(43) Date of publication:
**01.03.2017 Bulletin 2017/09**

(51) Int Cl.:
***G06F 19/00*** *(2011.01)*

(21) Application number: **16185092.0**

(22) Date of filing: **22.08.2016**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **24.08.2015 JP 2015165256**

(71) Applicant: **Panasonic Intellectual Property Management Co., Ltd.**
**Osaka-shi, Osaka 540-6207 (JP)**

(72) Inventors:
- **NAKADA, Toru**
  **Osaka-shi, Osaka 540-6207 (JP)**
- **SATO, Yoshikuni**
  **Osaka-shi, Osaka 540-6207 (JP)**

(74) Representative: **Grünecker Patent- und Rechtsanwälte**
**PartG mbB**
**Leopoldstraße 4**
**80802 München (DE)**

## (54) WALKING-LOAD-DEGREE CALCULATION APPARATUS, MAXIMUM-OXYGEN-CONSUMPTION CALCULATION APPARATUS, AND CONTROL METHOD

(57) A walking-load-degree calculation apparatus includes a rate obtainer that obtains a rate that is a heart rate or a pulse rate of a user, a walking speed obtainer that obtains one or more walking speeds of the user during a walk, a weight obtainer that obtains a weight of the user, and a walking-load-degree calculator that calculates a walking load degree that is an indicator of cardiopulmonary exercise of the user during the walk.

FIG. 1

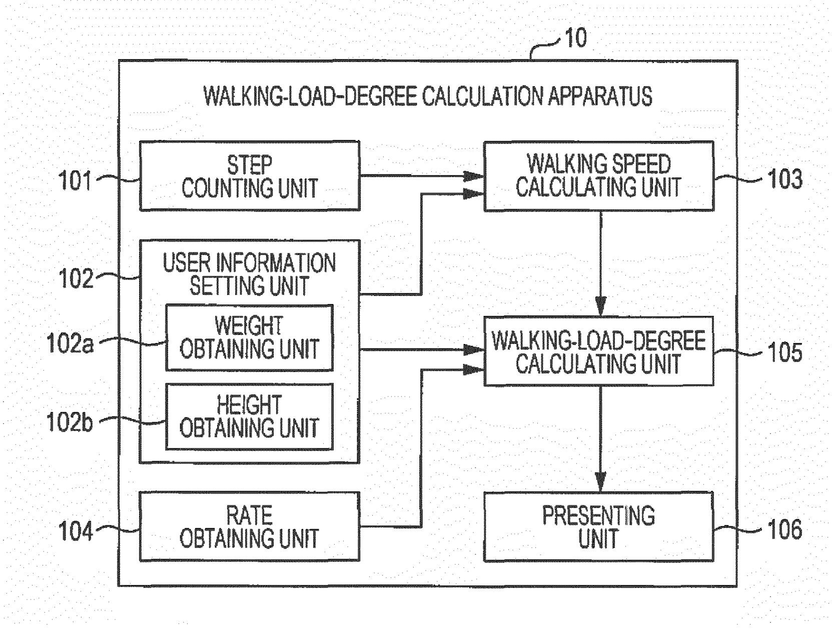


EP 3 136 269 A1

## Description

BACKGROUND

1. Technical Field

The present disclosure relates to a walking-load-degree calculation apparatus that measures a heart rate and a walking speed of a person during a walk and calculates a walking load degree of the person on the basis of the measured heart rate and walking speed and to a maximum-oxygen-consumption calculation apparatus and a control method.

2. Description of the Related Art

The number of people who manage their health through easy-to-do exercise such as walking and jogging has recently been increasing. Chest-strap-type heart rate monitors and wristband-type pulsimeters (hereinafter, collectively referred to as "rate monitors") are widely used by such people to monitor an exercise load during exercise. Such rate monitors allow users to check their heart/pulse rates detected in real time and to adjust the exercise intensity to an appropriate level (see, for example, Japanese Unexamined Patent Application Publication No. 7-213499).

It is, however, difficult to accurately estimate a walking load degree of a person during a walk by using the rate monitors. Herein, the "walking load degree" is an indicator indicating a physiological body load for a person during a walk as well as an indicator indicating a physical body load for the person during the walk. Examples of the indicator indicating the physiological body load include the heat rate, the pulse rate, or the like, whereas examples of the indicator indicating the physical body load include an indicator indicating the exercise intensity, for example, a walking speed. Accordingly, the rate monitors estimate only the physiological body load for a person during a walk and are incapable of estimating the physical body load.

SUMMARY

One non-limiting and exemplary embodiment provides a walking-load-degree calculation apparatus capable of accurately estimating a walking load degree of a person during a walk.

In one general aspect, the techniques disclosed here feature a walking-load-degree calculation apparatus including a rate obtainer, a walking speed obtainer, a weight obtainer, and a walking-load-degree calculator. The rate obtainer obtains a rate of a user. The rate is a heart rate or a pulse rate of the user. The walking speed obtainer obtains one or more walking speeds of the user during a walk. The weight obtainer obtains a weight of the user. The walking-load-degree calculator calculates a walking load degree of the user. The walking load degree is an indicator of cardiopulmonary exercise of the user during the walk and is defined as

$$\tau = \frac{\sum_{t=1}^{n} (HRw(t) - HRr)}{\sum_{t=1}^{n} m \cdot v(t)^2},$$

where

τ denotes the walking load degree, HRw(t) denotes a rate of the user obtained by the rate obtainer for a t-th period during the walk, a period of the walk being divided into n periods including the t-th period, each of the n periods being a predetermined period of time, t being an integer of 1 or greater, and n being an integer of 1 or greater, HRr denotes a resting rate of the user obtained by the rate obtainer for the predetermined period of time, m denotes the weight of the user obtained by the weight obtainer, and v(t) denotes a walking speed of the user obtained by the walking speed obtainer for the t-th period among the one or more walking speeds.

According to the general aspect, a walking load degree, which is an indicator of a physiological body load for a person during a walk as well as a indicator of a physical body load for the person during the walk, is successfully estimated accurately.

It should be noted that general or specific embodiments may be implemented as a method, a system, an integrated circuit, a computer program, a non-transitory computer-readable recording medium, or any selective combination thereof. Examples of the non-transitory computer-readable recording medium include a nonvolatile recording medium, such as a Compact Disc-Read Only Memory (CD-ROM).

Additional benefits and advantages of the disclosed embodiments will become apparent from the specification and drawings. The benefits and/or advantages may be individually obtained by the various embodiments and features of the specification and drawings, which need not all be provided in order to obtain one or more of such benefits and/or advantages.

BRIEF DESCRIPTION OF THE DRAWINGS

Fig. 1 is a block diagram illustrating a functional configuration of a walking-load-degree calculation apparatus according to a first embodiment of the present disclosure;
Fig. 2 is a diagram for describing a method for calculating each term of Equation 1 used for calculating a walking load degree;
Fig. 3 is a graph showing how the walking load degree changes for individual endurance-level groups;
Fig. 4 is a flowchart illustrating a preprocessing operation performed by the walking-load-degree calculation apparatus;
Fig. 5 is a flowchart illustrating a measurement processing operation performed by the walking-load-degree calculation apparatus;
Fig. 6 is a diagram illustrating an example of an image displayed on a screen of a presenting unit of a wristband-type walking-load-degree calculation apparatus;
Fig. 7 is a graph illustrating a relationship between the walking load degree and the maximum oxygen consumption;
Fig. 8 is a block diagram illustrating a functional configuration of a maximum-oxygen-consumption calculation apparatus according to a second embodiment of the present disclosure;
Fig. 9 is a block diagram illustrating a functional configuration of a walking-load-degree calculation apparatus according to a third embodiment of the present disclosure; and
Fig. 10 is a flowchart illustrating a processing operation performed by the walking-load-degree calculation apparatus.

DETAILED DESCRIPTION

A walking-load-degree calculation apparatus according to a first aspect of the present disclosure includes: a rate obtainer that obtains a rate of a user, the rate being a heart rate or a pulse rate of the user; a walking speed obtainer that obtains one or more walking speeds of the user during a walk; a weight obtainer that obtains a weight of the user; and a walking-load-degree calculator that calculates a walking load degree of the user, the walking load degree being an indicator of cardiopulmonary exercise of the user during the walk and being defined as

$$\tau = \frac{\sum_{t=1}^{n}(HRw(t) - HRr)}{\sum_{t=1}^{n} m \cdot v(t)^2},$$

where

$\tau$ denotes the walking load degree,
HRw(t) denotes a rate of the user obtained by the rate obtainer for a t-th period during the walk, a period of the walk being divided into n periods including the t-th period, each of the n periods being a predetermined period of time, t being an integer of 1 or greater, and n being an integer of 1 or greater,
HRr denotes a resting rate of the user obtained by the rate obtainer for the predetermined period of time,
m denotes the weight of the user obtained by the weight obtainer, and
v(t) denotes a walking speed of the user obtained by the walking speed obtainer for the t-th period among the one or more walking speeds.

With this configuration, a change in the body condition during walking exercise can be objectively and easily grasped. Accordingly, a walking load degree, which is an indicator of a physiological body load for a person during a walk as well as a physical body load for the person during the walk, can be easily calculated.

In addition, for example, the walking-load-degree calculation apparatus may further include a presenter that presents the walking load degree calculated by the walking-load-degree calculator to the user.

With this configuration, the user can be informed of the calculated walking load degree.

In addition, for example, the walking-load-degree calculation apparatus may further include: a height obtainer that obtains a height of the user; a step counter that counts steps of the user during the walk; and a walking speed calculator that calculates the one or more walking speeds of the user, wherein the walking speed calculator may calculate the number of steps counted by the step counter for the t-th period, calculate a stride length of the user during the walk by using the height obtained by the height obtainer and the number of steps for the t-th period, and calculate the walking speed for the t-th period based on a product of the number of steps for the t-th period and the stride length, and wherein the walking speed obtainer may obtain the one or more walking speeds calculated by the walking speed calculator.

With this configuration, the user's walking speed can be calculated by just providing a simple sensor such as a step counter that counts steps of the user if a height of the user is obtained. Consequently, the walking-load-degree calculation apparatus having a simple configuration can be implemented.

In addition, a maximum-oxygen-consumption calculation apparatus according to another aspect of the present disclosure includes: the walking-load-degree calculation apparatus described above; and a maximum-oxygen-consumption calculator that calculates a maximum oxygen consumption of the user by using a relationship calculated in advance for a walking load degree and a maximum oxygen consumption and using the walking load degree calculated by the walking-load-degree calculation apparatus.

With this configuration, since a walking load degree, which is an indicator of a physiological body load for a person during a walk as well as a physical body load for the person during the walk, can be easily calculated, the maximum oxygen consumption of the walking person can be easily calculated.

In addition, the maximum-oxygen-consumption calculation apparatus may further include a presenter that presents the maximum oxygen consumption calculated by the maximum-oxygen-consumption calculator to the user.

With this configuration, the user can be informed of the calculated maximum oxygen consumption.

A walking-load-degree calculation apparatus according to still another aspect of the present disclosure includes: a rate sensor that obtains a rate of a user for a predetermined period of time during a walk, the rate being a heart rate or a pulse rate of the user; and a calculation circuit that calculates a walking load degree of the user, the walking load degree being an indicator of cardiopulmonary exercise of the user during the walk, wherein the calculation circuit (a) obtains a weight of the user, a walking speed of the user for the predetermined period of time, a resting rate of the user, and the rate of the user for the predetermined period of time during the walk, (b) calculates an exercise energy consumption for the predetermined period of time on the basis of the weight of the user and the walking speed of the user for the predetermined period of time, (c) calculates an increase in the rate of the user for the predetermined period of time during the walk from the resting rate of the user on the basis of the resting rate of the user and the rate of the user obtained for the predetermined period of time during the walk, and (d) calculates, as the walking load degree, a ratio of the increase in the rate of the user to the exercise energy consumption for the predetermined period of time.

The walking-load-degree calculation apparatus may further include a presenter that presents the ratio of the increase in the rate of the user to the exercise energy consumption for the predetermined period of time.

It should be noted that these general or specific aspects may be implemented as a method, a system, an integrated circuit, a computer program, or a non-transitory computer-readable recording medium such as CD-ROM, or any selective combination thereof.

Embodiments of the present disclosure will be described below with reference to the accompanying drawings.

## First Embodiment

### Configuration

Fig. 1 is a block diagram illustrating a functional configuration of a walking-load-degree calculation apparatus 10 according to a first embodiment of the present disclosure.

The walking-load-degree calculation apparatus 10 includes a step counting unit 101, a user information setting unit 102, a walking speed calculating unit 103, a rate obtaining unit 104, a walking-load-degree calculating unit 105, and a presenting unit 106. The walking-load-degree calculation apparatus 10 is implemented as a chest-strap-type or wristband-type user wearable device.

### Step Counting Unit 101

The step counting unit 101 is a unit that counts steps during walking exercise, which is a fundamental function of a pedometer. The step counting unit 101 may be implemented by a pendulum step sensor or an accelerometer step sensor. A signal output as a result of the step sensor detecting each step is amplified by an amplification circuit and is converted into a digital signal by an analog-to-digital (A/D) conversion circuit. By using such an existing technique, the step counting unit 101 having a high accuracy within an error defined by a standard (± 3% according to the Japanese Industrial Standards (JIS)) can be implemented.

In addition, the step counting unit 101 determines that a time point at which the first step is detected by the step sensor is the start of walking. The step counting unit 101 also determines that a time point at which a predetermined period (e.g., 10 seconds) passes since the step sensor stops detecting a step is the end of walking.

User Information Setting Unit 102

The user information setting unit 102 includes a weight obtaining unit 102a that obtains a weight of a user who utilizes the walking-load-degree calculation apparatus 10 and a height obtaining unit 102b that obtains a height of the user. The user information setting unit 102 is implemented by a central processing unit (CPU), a memory storing a program, an input device (such as buttons, keys, or a touch panel), etc. The user information setting unit 102 may be implemented by a communication unit, such as a communication module, that obtains such information from an external terminal or a server by communication using a mobile phone network, Wireless Fidelity (Wi-Fi), Bluetooth (registered trademark), or the like. The obtained weight and height are stored in a storage unit (not illustrated). The storage unit is implemented by, for example, a storage device such as registers or a cache of the CPU, a random access memory (RAM), or a read-only memory (ROM). Note that the user information setting unit 102 may obtain ID information of the user. For example, the user information setting unit 102 may refer to an external database by using the ID information of the user and obtain the height and weight of the user. The database stores ID information of each user and a height and a weight of the user associated with the ID information.

Walking Speed Calculating Unit 103

The walking speed calculating unit 103 calculates a walking pitch (steps/minute) per unit time (e.g., 1 minute) from the step count indicating the number of steps counted by the step counting unit 101. Specifically, the walking speed calculating unit 103 includes a time measuring unit and calculates the walking pitch on the basis of the step count obtained from the step counting unit 101 every minute, for example. The obtained step count may be a cumulative value from when step counting has been started or an accumulated value from when the step count has been obtained the last time. If the step count is a cumulative value, the walking speed calculating unit 103 calculates the walking pitch by subtracting the cumulative value obtained the last time from the cumulative value obtained this time. If the step count is an accumulated value from when the step count has been obtained the last time, the walking speed calculating unit 103 determines the accumulated value as the walking pitch. Note that if the walking speed calculating unit 103 obtains the step count every predetermined period (minutes) instead of every minute, the walking speed calculating unit 103 calculates the walking pitch by dividing the obtained step count by the predetermined period.

The walking speed calculating unit 103 then calculates a stride length (m) from the user's height obtained by the user information setting unit 102. Since the stride length correlates to the height, the stride length can be generally determined using "stride length = height x 0.45" in the case of walking at ordinary speed. In addition, the stride length is known to become wider as the walking pitch increases and to become narrower as the walking pitch decreases. Accordingly, for example, in the case of walking at low speed for which the walking pitch is 110 (steps/minute) or less, "stride length = height x 0.40" is used. In the case of walking at ordinary speed for which the walking pitch is 110 to 130 (steps/minute), "stride length = height x 0.45" is used. In the case of walking at high speed for which the walking pitch is 130 (steps/minute) or greater, "stride length = height x 0.50" is used. Increasing a coefficient by which the height is multiplied to calculate the stride length as the walking pitch increases allows the stride length to be determined more accurately.

The walking speed calculating unit 103 may allow the user to set their standard stride length. In such a case, the user may calculate the stride length of one step by counting steps by walking a predetermined distance (e.g., 10 m) at ordinary speed and may set this stride length as a stride length for the case of walking at ordinary speed for which the walking pitch is 110 to 130 (steps/minute). Further, the user may determine the stride length in accordance with the walking pitch such that the stride length is 8/9 of the ordinary stride length in the case of walking at low speed for which the walking pitch is 110 (steps/minute) or less and that the stride length is 10/9 of the ordinary stride length in the case of walking at high speed for which the walking pitch is 130 (steps/minute) or greater.

Lastly, the walking speed calculating unit 103 calculates a walking speed of a walk for a unit time by multiplying the above-described stride length and the above-described walking pitch.

The walking speed calculating unit 103 is implemented by a CPU, a memory storing a program, etc. For example, the walking speed calculating unit 103 calculates a walking speed of a user as a result of the CPU executing the program stored in the memory. The walking speed calculating unit 103 may store the calculated walking speed in the memory.

Note that the walking speed calculating unit 103 may obtain position information determined by a known positioning system, such as Global Positioning System (GPS), and calculate a walking speed on the basis of the position information, in addition to calculating the walking speed from the walking pitch as described above. For example, position information of the user may be obtained using a GPS receiver included in the user wearable device every predetermined period while the user is walking, and a distance which the user has walked may be calculated by linking positions indicated by the position information by a line while the user is walking. A value obtained by dividing the walking distance by a time taken for the walk can be calculated as the walking speed. In this case, the walking speed calculating unit 103 is implemented by a GPS receiver, a CPU, a memory storing a program, etc. In this case, the walking speed calculating unit 103 does not need step count information since it is capable of calculating the walking speed on the basis of information obtained by the positioning system.

Note that the walking speed calculating unit 103 may calculate the walking speed on the basis of a walking distance set by the user in advance using the user wearable device. For example, the walking speed calculating unit 103 may allow the user to set a walking distance in advance and allow the user to explicitly input the start timing of walking and the end timing of walking. Then, the walking speed calculating unit 103 may calculate the walking speed by dividing the set walking distance by a period from the start of walking to the end of walking.

Rate Obtaining Unit 104

The rate obtaining unit 104 measures a user's heart/pulse rate to obtain the user's heart/pulse rate. A known heart/pulse rate sensor (also referred to as a rate sensor) capable of obtaining a user's heart/pulse rate is used. An example of the rate obtaining unit 104 is a measurement sensor put on the user's chest or wrist. The measurement sensor put on the chest (also referred to as a chest-strap-type measurement sensor) is fixed to the user's chest using a strap and measures the user's heart rate. The measurement sensor put on the wrist (also referred to as a wristband-type measurement sensor) is fixed to the user's wrist using a band and measures the pulse rate at the wrist. Note that the rate obtaining unit 104 may obtain the heart/pulse rate data without measuring the user's resting heart/pulse rate. In this case, the walking-load-degree calculating unit 105 (described later) obtains the user's resting heart/pulse rate stored in the memory. Specifically, the walking-load-degree calculating unit 105 obtains the resting heart/pulse rate stored in the memory with reference to the user information.

An example of the rate obtaining unit 104 is a heart rate sensor that measures the heart rate. The heart rate sensor includes a casing, a measurement circuit housed inside the casing, and a plurality of electrodes. The measurement circuit is electrically connected to the plurality of electrodes. The plurality of electrodes are placed on the user's chest or a portion near the chest. The measurement circuit measures a cardio activity potential signal between a plurality of electrodes. Note that the casing may be placed on the user's chest or a portion near the chest. The rate obtaining unit 104 may also include various circuits, such as an amplification circuit that amplifies the obtained cardio activity potential signal, a low-pass filter circuit that removes noise from the cardio activity potential signal, and an A/D conversion circuit that converts the cardio activity potential signal into a digital signal. The rate obtaining unit 104 includes the measurement circuit that measures the heart rate by detecting cardio activity potential pulses from the digital signal resulting from the A/D conversion and counting the cardio activity potential pulses for unit time (e.g., 1 minute). Alternatively, the rate obtaining unit 104 may measure a heart rate per unit time (e.g., 1 minute) by measuring a time interval (R-R interval) between cardio activity potential pulses and dividing the unit time by the measured time interval (R-R interval). The rate obtaining unit 104 is implemented by, for example, a CPU, a memory storing a program, etc. The rate obtaining unit 104 obtains the heart/pulse rate as a result of the CPU executing the program stored in the memory. The rate obtaining unit 104 may store the measured heart/pulse rate in the memory.

Another example of the rate obtaining unit 104 is a pulse sensor that measures a pulse rate. As the rate obtaining unit 104, a known pulse sensor including a photoelectric pulse sensor is used. The pulse sensor is placed on a body portion where the pulse is detectable, such as the wrist or the neck, and measures the user's pulse rate. The photoelectric pulse sensor includes a light-emitting element (e.g., infrared light-emitting diode (LED)) and a photosensitive element (e.g., phototransistor) that detects reflected light of infrared emitted from the light-emitting element and measures a pulse wave that represents a change in the blood flow. The rate obtaining unit 104 may further include various circuits, such as an amplification circuit that amplifies a pulse wave signal obtained by measuring the pulse wave, a low-pass filter circuit that removes noise from the pulse wave signal, and an A/D conversion circuit that converts the pulse wave signal into a digital signal. The rate obtaining unit 104 measures the pulse rate per unit time (e.g., 1 minute) from the digital signal resulting from A/D conversion.

Note that measurement of the heart/pulse rate may be started and ended in response to a user operation. Alternatively, in the case of walking, measurement of the heart/pulse rate may be started upon the step counting unit 101 detecting walking and may be ended upon the step counting unit 101 stopping detecting walking.

Walking-Load-Degree Calculating Unit 105

The walking-load-degree calculating unit 105 calculates a walking load degree by using the user's weight, the user's walking speed during a walk, the user's resting heart/pulse rate, and the user's heart/pulse rate during the walk. For example, the walking-load-degree calculating unit 105 obtains the user's weight, the user's walking speed during a walk, the user's resting heart/pulse rate, and the user's heart/pulse rate during the walk by referring to the memory. The memory referred to at that time is not limited to one memory, and a plurality of memories may be referred to. Note that the user's resting heart/pulse rate is not limited to a value obtained by the rate obtaining unit 104.

The walking load degree is calculated using Equation 1 below.

$$\tau = \frac{\sum_{t=1}^{n}(HRw(t) - HRr)}{\sum_{t=1}^{n} m \cdot v(t)^2} \quad \text{(Equation 1)}$$

Fig. 2 is a diagram for describing a method for calculating each term of Equation 1 used for calculating the walking load degree.

In Equation 1, HRw(t) denotes a heart/pulse rate obtained by the rate obtaining unit 104 for a t-th period (where t is an integer of 1 or greater) during a walk. The t-th period is a predetermined period of time. Specifically, as illustrated in Fig. 2, a first period is a period for t = 1. For example, if the predetermined period of time is 1 minute, the first period is a period from when walking is started to when the first one minute passes. Note that the predetermined period of time need not necessarily be 1 minute and may be another period of time, such as 30 seconds or 2 minutes. In addition, a second period is a period for t = 2 and is a period from 1 minute after the start of walking to 2 minutes after the start of walking. In other words, the t-th period is a t-th period that has elapsed from the start of walking among a plurality of periods each of which is the predetermined period of time. The plurality of periods are all equal and are the predetermined period of time.

That is, HRw(1) denotes a heart/pulse rate measured in the first period. Specifically, HRw(1) denotes the number of heartbeats counted for the first period, i.e., 1 minute. HRw(2) denotes a heart/pulse rate measured in the second period. Specifically, HRw(2) denotes the number of heartbeats counted for the second period, i.e., 1 minute. Note that a measurement result obtained for a period shorter than the predetermined period of time may be used to estimate a measurement result supposed to be obtained for the predetermined period of time, and the estimated result may be used as the heart/pulse rate obtained for the predetermined period during a walk. Herein, the expression "during a walk" indicates a period over which the step sensor of the step counting unit 101 keeps detecting a signal. It is assumed that the user is walking if an interval at which the step sensor detects the signal is equal to a predetermined period (e.g., 1 second) or less.

HRr denotes the resting heart/pulse rate per predetermined period of time. Specifically, HRr denotes an average heart/pulse rate measured in a predetermined period of time during a rest. The predetermined period of time is equal to that of the t-th period, i.e., 1 minute. Herein, the expression "during a rest" indicates a predetermined period for which there is no physiological and physical change of the user. The resting heart/pulse rate can be measured by the user in advance using the user wearable device. Specifically, the user may measure the heart/pulse rate per unit time by using the user wearable device after keeping a sitting position for a predetermined period of time (e.g., 1 minute).

m denotes the user's weight obtained by the weight obtaining unit 102a.

v(t) denotes a walking speed calculated by the walking speed calculating unit 103 for the t-th period during the walk. The t-th period is the same as described above.

n is an integer of 1 or greater. Note that Fig. 2 illustrates a case where n > 4; however, Fig. 2 is merely an example and n may be any integer that is greater than or equal to 1.

As illustrated in Fig. 2(b), an increase in the heart/pulse rate is calculated for each period by subtracting the resting heart/pulse rate (Fig. 2(a)) from a corresponding one of the plurality of heart/pulse rates measured in the plurality of periods during the walk. Then, the sum of the increases in the heart/pulse rate for the respective periods from the first period to the n-th period is calculated. In this way, the numerator of Equation 1 is calculated.

In addition, as illustrated in Fig. 2(c), an exercise energy consumption is calculated for each period by using the user's weight and a corresponding one of the plurality of walking speeds measured for a corresponding one of the plurality of periods. Then, the sum of the exercise energy consumptions for the respective periods from the first period to the n-th period is calculated. In this way, the denominator of Equation 1 is calculated.

Lastly, the walking load degree for the t-th period is calculated by dividing the numerator of Equation 1 determined in Fig. 2(b) by the denominator of Equation 1 determined in Fig. 2(c). That is, the walking load degree denotes a heart rate increased due to walking exercise relative to an amount of energy consumed by a walk up to the t-th period. In other words, the walking load degree is denoted as a ratio between (an amount of energy consumed by exercise for a predetermined period) and (a total increase in the heart/pulse rate for the predetermined period). That is, the walking load degree can be denoted not only as (the amount of energy consumed by exercise for the predetermined period)/(the total increase in the heart/pulse rate for the predetermined period) but also (the total increase in the heart/pulse rate for the predetermined period)/(the amount of energy consumed by exercise for the predetermined period).
If there is only one period, the sums respectively calculated in Fig. 2(b) and (c) are results calculated for the one period. That is, the walking load degree may be a value obtained by dividing the increase in the heart/pulse rate calculated for the one period by the exercise energy consumption for the one period.

Walking Load Degree

Now, a result of an experiment regarding the walking load degree carried out by the inventors will be described. The inventors confirmed based on the experiment described below that the walking load degree represents a user's walking function and a change in the state of the walking function. Details of the experiment will be described below.
24 subjects, 13 males and 11 females, whose average age was 68.0 $\pm$ 5.0, were subjected to two tests (first and second tests) described below.
The first test was a 6-minute walk test, which will be described below.
During the 6-minute walk test, the resting heart rate of each subject was measured using a heart rate sensor put on the chest of the subject who had been in a lying posture before the start of the test for a sufficient rest. Then, the subject was caused to walk a 30-meter straight course in both ways for 6 minutes with the heart rate sensor put on, and a recording person recorded a walking distance every minute and measured the heart rate of the walking subject every minute. At that time, the subject was instructed to walk as fast as possible within their safe limits.
The second test was a cardiopulmonary exercise test (CPX), which will be described below.
The cardiopulmonary exercise test is a test for measuring the maximum oxygen consumption, which is the most typical indicator of endurance. During the cardiopulmonary exercise test, the maximum oxygen consumption of each subject was measured under a Ramp protocol using a cycle ergometer (AEROBIKE75XLII, Combi Wellness) and an expiration gas analyzer (AE-300S, Minato Medical Science Co., Ltd).
A method for analyzing the results of these two tests will now be described. The subjects were classified into three groups (a high-endurance-level group, an intermediate-endurance-level group, and a low-endurance-level group) of 8 people each in descending order of the maximum oxygen consumption based on the maximum oxygen consumption measured during the cardiopulmonary exercise test. Then, for each of the three groups, an average of the walking load degrees of the 6-minute walk test was calculated.
Fig. 3 is a diagram illustrating how the walking load degree changes every minute for each of the endurance levels.
The horizontal axis of Fig. 3 denotes time (minute), and the vertical axis denotes the walking load degree (beats/J) determined using Equation 1. As illustrated in Fig. 3, a solid line at the lowermost position denotes the change for the high-endurance-level group, a dashed line in the middle denotes the change for the intermediate-endurance-level group, and a dotted line at the uppermost position denotes the change for the low-endurance-level group.
The graph of Fig. 3 indicates that the walking load degree changes at higher values for the group with a lower endurance level. The graph also indicates that the increase rate of the walking load degree is larger for the group with a lower endurance level.
A consideration by the inventors about the characteristics above will now be described.
It is considered that walking exercise applies a higher load on a group with a lower endurance level and the walking load degree is high for the subjects belonging to such a group. It can be considered that the fact that the walking load degree changes at higher values for the group with a lower endurance level according to the result of the experiment represents this influence. It is also considered that an increase in the walking load degree is also larger for the group with a lower endurance level. It can be considered that the fact that the increase rate of the walking load degree is higher for the group with a lower endurance level represents this influence.
Accordingly, the walking-load-degree calculating unit 105 calculates the walking load degree, which is an indicator of the load applied by walking. In this way, a change in the body condition during walking exercise can be objectively and easily grasped.

Presenting Unit 106

The presenting unit 106 displays, on a screen included in the walking-load-degree calculation apparatus 10, the value of the walking load degree calculated by the walking-load-degree calculating unit 105 or information indicating a change in the walking load degree with time. In this case, the presenting unit 106 is implemented by a display device, such as a liquid crystal display capable of displaying the information on the screen.

The presenting unit 106 need not necessarily include a screen. In this case, the presenting unit 106 may display the walking load degree using an external display device provided outside the walking-load-degree calculation apparatus 10. In addition, the presenting unit 106 may be implemented by a speaker that outputs the information as sound in addition to the display device that displays the information on the screen.

Operations

Various operations performed by the walking-load-degree calculation apparatus 10 thus configured will be described next.

Figs. 4 and 5 are flowcharts illustrating processing operations (control method) performed by the walking-load-degree calculation apparatus 10 according to the first embodiment.

Fig. 4 is a flowchart illustrating a preprocessing operation performed by the walking-load-degree calculation apparatus 10.

In step S301, the user information setting unit 102 accepts information on the user's height and weight and stores the accepted information as user information in a memory (also referred to as user information accepting processing).

Then, in step S302, the rate obtaining unit 104 measures the resting heart/pulse rate of the user and stores the resting heart/pulse rate in the memory

(also referred to as resting heart/pulse rate obtaining processing). Note that this resting heart/pulse rate obtaining processing is performed after the user is confirmed to be in the resting state as described above.

Note that the order of steps S301 and S302 may be opposite.

Fig. 5 is a flowchart illustrating an operation of a walking-load-degree calculation process.

The walking-load-degree calculation process starts upon detection of a user operation indicating the start of walking or upon detection of the start of walking by the step counting unit 101.

First, in step S401, the step counting unit 101 counts steps of the user during walking exercise (also referred to as step counting processing).

Then, in step S402, the walking speed calculating unit 103 calculates, as a walking pitch, the number of steps per unit time on the basis of the step count obtained by the step counting unit 101. The walking speed calculating unit 103 calculates a stride length during a walk by using the user's height accepted by the user information setting unit 102 and the waking pitch. The walking speed calculating unit 103 multiplies the stride length by the walking pitch, and calculates the walking speed per unit time (also referred to as walking speed calculation processing).

In step S403, the rate obtaining unit 104 measures the heart/pulse rate of the user per unit time during the walk (also referred to as walking heart/pulse rate obtaining processing), in parallel to steps S401 and S402.

In step S404, the walking-load-degree calculating unit 105 calculates the sum of the exercise energy consumptions for respective walking periods from the first period to the n-th period, by using the walking speeds calculated by the walking speed calculating unit 103 and the user's weight accepted by the user information setting unit 102. Then, the walking-load-degree calculating unit 105 subtracts the resting heart/pulse rate from each of the walking heart/pulse rates obtained by the rate obtaining unit 104 for the respective periods from the first period to the n-th period and calculates the sum of the increases in the heart/pulse rate for the individual periods from the first period to the n-th period. The walking-load-degree calculating unit 105 calculates, as the walking load degree, a value obtained by dividing the sum of the increases in the heart/pulse rate by the sum of exercise energy consumptions (also referred to as walking-load-degree calculation processing).

In step S405, the presenting unit 106 presents the walking load degree calculated by the walking-load-degree calculating unit 105 to the user (also referred to as presenting processing). An example of the presenting unit 106 is a display or a speaker. If the presenting unit 106 is a display, a graph indicating how the calculated walking load degree (beats/J) changes every minute may be displayed on the screen of the display as illustrated in Fig. 6. If the presenting unit 106 is a speaker, the walking load degree is presented by sound. For example, the walking load degree is presented every minute together with the measurement time such that (1 minute, 0.48), (2 minutes, 0.598), (3 minutes, 0.698), (4 minutes, 0.695), and (5 minutes, 0.695).

Fig. 6 is a diagram illustrating an example of an image displayed on the screen of a presenting unit of a wristband-type walking-load-degree calculation apparatus.

Then, the walking-load-degree calculation apparatus 10 determines whether the user has finished walking on the basis of whether the user has performed an operation indicating the end of walking or the step counting unit 101 has detected the end of walking (step S406). If it is determined that the walking has been finished (YES in S406), the walking-load-degree calculation apparatus 10 ends the walking-load-degree calculation process. If it is determined that walking has not been finished (NO in S406), the walking-load-degree calculation apparatus 10 performs the walking-load-degree calculation process again.

Advantageous Effects, etc.

As described above, according to the first embodiment, the walking-load-degree calculation apparatus 10 successfully calculates the walking load degree using Equation 1 from the user's weight, the walking speeds obtained during a walk, the user's resting heart/pulse rate, and the user's heart/pulse rates obtained during the walk. This consequently allows a change in the body condition during walking exercise to be objectively and easily grasped. Accordingly, the walking load degree, which is an indicator of a physiological body load for a person during a walk as well as an indicator of a physical body load for the person during the walk, can be easily calculated.

Second Embodiment

A second embodiment will be specifically described next. In the second embodiment, a maximum-oxygen-consumption calculation apparatus that estimates the maximum oxygen consumption (VO2max), which is the most typical indicator of endurance, by using a walking load degree will be described.

As a method for indirectly estimating the maximum power consumption according to the related art, the Astrand-Ryhming nomogram is known (see Astrand PO, Ryhming I., "A nomogram for calculation of aerobic capacity from pulse rate during submaximal work", J Appl Physiol 1954, 7, pp.218-221 (hereinafter, referred to as NPL 1)). In this method, subjects are caused to do exercise at a constant load using am electrically controlled cycle ergometer for 6 minutes, and the maximum oxygen consumption is estimated from a relationship between the exercise load (workload) and the heart rate using the nomogram created by them. This method, however, requires an exercise load that makes the heart rate 120 bpm or higher, possibly causing a risk for elderly or sick people. Further, people who are not used to ride bicycles may experience local muscle fatigue soon, leading to underestimation of endurance.

In addition, a maximum-oxygen-consumption estimation method using a wristband-type mobile terminal is known (see Japanese Patent No. 3608204). In this method, an exercise load (weight x running speed) and the heart rate during running are calculated using a wristband-type mobile terminal, and the maximum oxygen consumption is estimated using the Astrand-Ryhming nomogram of NPL 1. This method, however, assumes a steady state during running, and it is difficult to obtain a highly accurate result when the method is applied to low-load exercise, such as walking. Therefore, this method requires elderly or sick people to run and thus is difficult to be applied to elderly or sick people.

Accordingly, in the second embodiment, a method for estimating the maximum oxygen consumption, which is the most typical indicator of endurance, from low-load exercise such as walking will be described.

The inventors found out that there is a high correlation between the maximum oxygen consumption and the walking load degree described in the first embodiment.

Now, a result of an experiment carried out by the inventors for the relationship between the maximum oxygen consumption and the walking load degree will be described. In the experiment, two tests were carried out. The content and the subjects of the tests were the same as those described in the first embodiment.

Fig. 7 illustrates a relationship between the walking load degree and the maximum oxygen consumption of each subject during a 6-minute walk. The horizontal axis denotes the walking load degree (beats/J) of the 6-minute walk determined using Equation 1, and the vertical axis denotes the maximum oxygen consumption (ml/minute).

Fig. 7 indicates that there is a high correlation between the walking load degree and the maximum oxygen consumption since the maximum oxygen consumption decreases as the walking load degree (the ratio of the increase in the heart/pulse rate from the resting heart rate obtained after a 6-minute rest to the 6-minute exercise energy consumption) increases. Specifically, a correlation coefficient r for "the walking load degree (the ratio of the increase in the heart/pulse rate from the resting heart rate obtained after a 6-minute rest to the 6-minute exercise energy consumption)" and "the maximum oxygen consumption" is -0.81.

A 6-minute walking distance of the 6-minute walk test is an indicator of a general simple endurance test for elderly people. The 6-minute walking distance and the maximum oxygen consumption have been reported to have a correlation (see Cahalin LP, et al., "The six-minute walk test predicts peak oxygen uptake and survival in patients with advanced heart failure", Chest., 1996, 110, pp. 325-332), and The Ministry of Education, Culture, Sports, Science and Technology has adopted the 6-minute walking distance as an indicator for evaluating endurance of elderly people in the new physical fitness test (see The Ministry of Education, Culture, Sports, Science and Technology, Shin Tairyoku Tesuto -Yuigina katuyo no tameni (New Physical Fitness Test -for the purpose of meaningful utilization), Gyosei, 2000).

The inventors carried out an experiment to examine the correlation between the 6-minute walking distance and the maximum oxygen consumption and obtained a result indicating that the correlation coefficient r is equal to 0.51. This experiment result suggests that the walking load degree is an extremely reliable indicator to estimate the maximum oxygen consumption, compared to the 6-minute walking distance.

Considerations about the above-described characteristics by the inventors are described below.

It is considered that since the 6-minute walking distance is affected by a psychological aspect that how hard the subject walks, the correlation coefficient decreases.

In addition, the Astrand-Ryhming nomogram is based on the assumption that the exercise load (workload) and the heart rate during the maximum work (e.g., running) and the oxygen consumption have a substantially linear relationship and that the maximum heart rate for people of substantially the same age is constant. However, the exercise load (workload) and the oxygen consumption do not necessarily have a linear relationship during a walk. In addition, variations in the motion ability among individuals are larger particularly for elderly people than young people, and the maximum heart rate is unlikely to be constant for people of substantially the same age.

It can be considered that the abovementioned variations are cancelled out by utilizing, instead of the exercise load (workload), a physical exercise energy consumption spent for walking for a predetermined (certain) period of time and the sum of increases in the heart rate caused by the walking during the predetermined period of time to calculate the walking load degree according to the embodiment of the present disclosure.

Accordingly, the use of the aforementioned relationship allows the maximum oxygen consumption to be estimated from the low-load exercise such as walking.

Configuration

Maximum-Oxygen-Consumption Estimating Unit 107

Fig. 8 is a block diagram illustrating a functional configuration of a maximum-oxygen-consumption calculation apparatus 20 according to the second embodiment.

The maximum-oxygen-consumption calculation apparatus 20 includes the step counting unit 101, the user information setting unit 102, the walking speed calculating unit 103, the rate obtaining unit 104, the walking-load-degree calculating unit 105, the presenting unit 106, and a maximum-oxygen-consumption estimating unit 107. In Fig. 8, components substantially the same as those illustrated in Fig. 1 are assigned the same reference signs, and a description thereof is omitted.

The maximum-oxygen-consumption estimating unit 107 estimates, using an estimation equation stored in advance, the maximum oxygen consumption from the walking load degree calculated by the walking-load-degree calculating unit 105. Note that the maximum-oxygen-consumption estimating unit 107 need not necessarily estimate the maximum oxygen consumption using the estimation equation stored in advance and may use a relationship between the walking load degree and the maximum oxygen consumption calculated in advance. That is, a table representing a relationship between the walking load degree and the maximum oxygen consumption may be used instead of the estimation equation. The maximum-oxygen-consumption estimating unit 107 is implemented by a CPU, a memory storing a program, etc.

The estimation equation is created based on data of the walking load degree and the maximum oxygen consumption collected in advance. The creation method may be based on linear regression analysis, logistic regression analysis, non-linear regression analysis such as support vector machine, or any other types of analysis. For example, if the creation method is based on linear regression analysis based on the experiment data, the maximum oxygen consumption can be estimated using an estimation equation below. Linear regression analysis is an analysis method for describing a correlation between a plurality of variables using a linear model. In this case, a slope a and an intercept b of the linear model y = ax + b are determined using sets of values of y (= maximum oxygen consumption) and x (= walking load degree), which are the experiment data. Specifically, the slope a and the intercept b can be determined using the least squares method for minimizing the sum of the squares of residues between the prediction value based on the linear model and each set of values of the experiment data. That is, the estimation equation can be expressed by Equation 2 below.

$$\text{Maximum oxygen consumption [ml/min]} = -1586.8 \times \text{Walking load degree} + 2503.8 \quad \text{(Equation 2)}$$

The presenting unit 106 displays the maximum oxygen consumption estimated by the maximum-oxygen-consumption estimating unit 107 instead of the walking load degree described in the first embodiment.

Advantageous Effects, etc.

As described above, according to the second embodiment, the maximum-oxygen-consumption calculation apparatus 20 successfully estimates the maximum oxygen consumption, which is the most typical indicator of endurance, by using the walking load degree of low-load exercise such as walking. As a result, measurement of the maximum oxygen consumption, which has been generally difficult for elderly or sick people, can be safely and easily carried out.

Third Embodiment

A third embodiment will be specifically described next. In the third embodiment, a walking-load-degree calculation apparatus 30 having the simplest configuration will be described.

Configuration

Fig. 9 is a block diagram illustrating a functional configuration of the walking-load-degree calculation apparatus 30 according to the third embodiment.

The walking-load-degree calculation apparatus 30 includes the weight obtaining unit 102a, the rate obtaining unit 104, a walking speed obtaining unit 303, and the walking-load-degree calculating unit 105. The walking-load-degree calculation apparatus 30 may further include the presenting unit 106. In Fig. 9, components substantially the same as those illustrated in Fig. 1 are assigned the same reference signs, and a description thereof is omitted.

The walking speed obtaining unit 303 may calculate the walking speed using the step count and the user's height as in the first embodiment. Alternatively, the walking speed obtaining unit 303 may obtain position information determined by a known positioning system, such as a GPS, and calculate the waking speed based on the position information. Alternatively, if the positioning system calculates the walking speed, the walking speed obtaining unit 303 may obtain the walking speed from the positioning system. Alternatively, the walking speed obtaining unit 303 may obtain the walking speed by calculating a traveling speed using an acceleration sensor. The weight obtaining unit 102a, the walking speed obtaining unit 303, and the walking-load-degree calculating unit 105 are also referred to as a calculation circuit.

Operations

Various operations performed by the walking-load-degree calculation apparatus 30 thus configured will be described next.

Fig. 10 is a flowchart illustrating a processing operation (control method) performed by the walking-load-degree calculation apparatus 30 according to the third embodiment.

The rate obtaining unit 104 or the walking-load-degree calculating unit 105 first obtains the user's resting heart/pulse rate (S901).

Then, the rate obtaining unit 104 obtains the user's heart/pulse rate during a walk (S902).

Then, the walking speed obtaining unit 303 obtains the user's walking speed (S903). Note that the user's heart/pulse rate and walking speed for a predetermined period during a walk are obtained in S902 and S903, respectively.

Then, the weight obtaining unit 102a obtains the user's weight (S904).

Lastly, the walking-load-degree calculating unit 105 calculates, using Equation 1, the walking load degree that is an indicator of the user's cardiopulmonary exercise during a walk (S905). If the walking-load-degree calculation apparatus 30 includes the presenting unit 106, the presenting unit 106 presents the walking load degree.

Advantageous Effects, etc.

As described above, according to the third embodiment, a change in the body condition during walking exercise can be objectively and easily grasped. Accordingly, the walking load degree, which is an indicator of a physiological body load for a person during a walk as well as an indicator of a physical body load for the person during the walk, can be easily calculated.

Note that the individual components of each of the embodiments described above may be configured by dedicated hardware or implemented by executing software programs suitable for the respective components. The individual components may be implemented as a result of a program executer, such as a CPU or a processor, reading and executing a software program stored on a recording medium, such as a hard disk or a semiconductor memory. The software that implements the walking-load-degree calculation apparatus and the like according to the embodiments described above is a control program described below.

Specifically, the control program is a control program executed in the walking-load-degree calculation apparatus, so that the rate obtaining unit of the walking-load-degree calculation apparatus obtains the resting rate, which is the user's heart or pulse rate during a rest, and obtains a walking rate, which is the user's heart or pulse rate during a walk, that the walking speed obtaining unit of the walking-load-degree calculation apparatus obtains the user's walking speed during the walk, that the weight obtaining unit of the walking-load-degree calculation apparatus obtains the user's weight, and that the walking-load-degree calculating unit of the walking-load-degree calculation apparatus calculates, using Equation 1 above, the walking load degree, which is an indicator of the user's cardiopulmonary exercise during the walk.

While the walking-load-degree calculation apparatus, the maximum-oxygen-consumption calculation apparatus, the control method, and the control program according to one or a plurality of aspects have been described above based on the embodiments, the present disclosure is not limited to these embodiments. Embodiments obtained by making alterations conceived by a person skilled in the art on the embodiments and embodiments configured by combining the components of different embodiments together may be within the scope of the one or plurality of aspects unless such embodiments depart from the spirits of the present disclosure.

The embodiments of the present disclosure are effectively used as apparatuses such as a walking-load-degree calculation apparatus capable of objectively and easily grasping a change in the body condition during walking exercise and a maximum-oxygen-consumption calculation apparatus capable of safely and easily grasping the maximum oxygen consumption during low-load exercise such as walking.

## Claims

1. A walking-load-degree calculation apparatus comprising:

   a rate obtainer that obtains a rate of a user, the rate being a heart rate or a pulse rate of the user;
   a walking speed obtainer that obtains one or more walking speeds of the user during a walk;
   a weight obtainer that obtains a weight of the user; and
   a walking-load-degree calculator that calculates a walking load degree of the user, the walking load degree being an indicator of cardiopulmonary exercise of the user during the walk and being defined as

$$\tau = \frac{\sum_{t=1}^{n}(HRw(t) - HRr)}{\sum_{t=1}^{n} m \cdot v(t)^2},$$

   where

   τ denotes the walking load degree,
   HRw(t) denotes a rate of the user obtained by the rate obtainer for a t-th period during the walk, a period of the walk being divided into n periods including the t-th period, each of the n periods being a predetermined period of time, t being an integer of 1 or greater, and n being an integer of 1 or greater,
   HRr denotes a resting rate of the user obtained by the rate obtainer for the predetermined period of time,
   m denotes the weight of the user obtained by the weight obtainer, and
   v(t) denotes a walking speed of the user obtained by the walking speed obtainer for the t-th period among the one or more walking speeds.

2. The walking-load-degree calculation apparatus according to Claim 1, further comprising:

   a presenter that presents the walking load degree calculated by the walking-load-degree calculator to the user.

3. The walking-load-degree calculation apparatus according to Claim 1, further comprising:

   a height obtainer that obtains a height of the user;
   a step counter that counts steps of the user during the walk; and
   a walking speed calculator that calculates the one or more walking speeds of the user,
   wherein the walking speed calculator

calculates the number of steps counted by the step counter for the t-th period,
calculates a stride length of the user during the walk by using the height obtained by the height obtainer and the number of steps for the t-th period, and
calculates the walking speed for the t-th period based on a product of the number of steps for the t-th period and the stride length, and

wherein the walking speed obtainer obtains the one or more walking speeds calculated by the walking speed calculator.

**4.** A maximum-oxygen-consumption calculation apparatus comprising:

the walking-load-degree calculation apparatus according to Claim 1; and
a maximum-oxygen-consumption calculator that calculates a maximum oxygen consumption of the user by using a relationship calculated in advance for a walking load degree and a maximum oxygen consumption and using the walking load degree calculated by the walking-load-degree calculation apparatus.

**5.** The maximum-oxygen-consumption calculation apparatus according to Claim 4, further comprising:

a presenter that presents the maximum oxygen consumption calculated by the maximum-oxygen-consumption calculator to the user.

**6.** A control method for a walking-load-degree calculation apparatus, comprising:

obtaining, with a rate obtainer included in the walking-load-degree calculation apparatus, a resting rate of a user, the resting rate being a heart rate or a pulse rate of the user during a rest;
obtaining, with the rate obtainer, a walking rate of the user, the walking rate being a heart rate or a pulse rate of the user during a walk;
obtaining, with a walking speed obtainer included in the walking-load-degree calculation apparatus, one or more walking speeds of the user during the walk;
obtaining, with a weight obtainer included in the walking-load-degree calculation apparatus, a weight of the user; and
calculating, with a walking-load-degree calculator included in the walking-load-degree calculation apparatus, a walking load degree of the user, the walking load degree being an indicator of cardiopulmonary exercise of the user during the walk and being defined as

$$\tau = \frac{\sum_{t=1}^{n}(HRw(t) - HRr)}{\sum_{t=1}^{n} m \cdot v(t)^2}$$

where

τ denotes the walking load degree,
HRw(t) denotes a walking rate of the user obtained by the rate obtainer for a t-th period, a period of the walk being divided into n periods including the t-th period, each of the n periods being a predetermined period of time, t being an integer of 1 or greater, and n being an integer of 1 or greater,
HRr denotes the resting rate of the user obtained by the rate obtainer for the predetermined period of time,
m denotes the weight of the user obtained by the weight obtainer, and
v(t) denotes a walking speed of the user obtained by the walking speed obtainer for the t-th period among the one or more walking speeds.

**7.** A walking-load-degree calculation apparatus comprising:

a rate sensor that obtains a rate of a user for a predetermined period of time during a walk, the rate being a heart rate or a pulse rate of the user; and
a calculation circuit that calculates a walking load degree of the user, the walking load degree being an indicator of cardiopulmonary exercise of the user during the walk, wherein
the calculation circuit

(a) obtains a weight of the user, a walking speed of the user for the predetermined period of time, a resting rate of the user, and the rate of the user for the predetermined period of time during the walk,
(b) calculates an exercise energy consumption for the predetermined period of time on the basis of the weight of the user and the walking speed of the user for the predetermined period of time,
(c) calculates an increase in the rate of the user for the predetermined period of time during the walk from the resting rate of the user on the basis of the resting rate of the user and the rate of the user obtained for the predetermined period of time during the walk, and
(d) calculates, as the walking load degree, a ratio of the increase in the rate of the user to the exercise energy consumption for the predetermined period of time.

**8.** The walking-load-degree calculation apparatus according to Claim 7, further comprising:

a presenter that presents the ratio of the increase in the rate of the user to the exercise energy consumption for the predetermined period of time.

FIG. 1

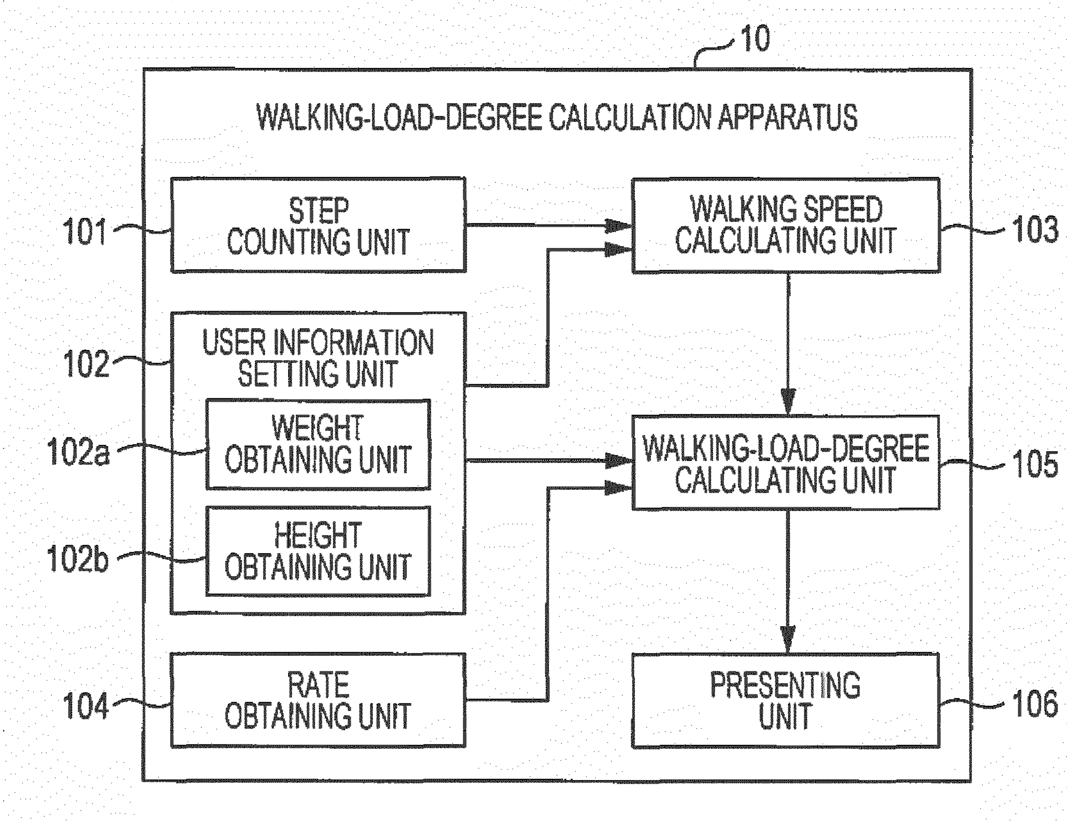
10
WALKING-LOAD-DEGREE CALCULATION APPARATUS
101
STEP COUNTING UNIT
102
USER INFORMATION SETTING UNIT
102a
WEIGHT OBTAINING UNIT
102b
HEIGHT OBTAINING UNIT
104
RATE OBTAINING UNIT
103
WALKING SPEED CALCULATING UNIT
105
WALKING-LOAD-DEGREE CALCULATING UNIT
106
PRESENTING UNIT

FIG. 2

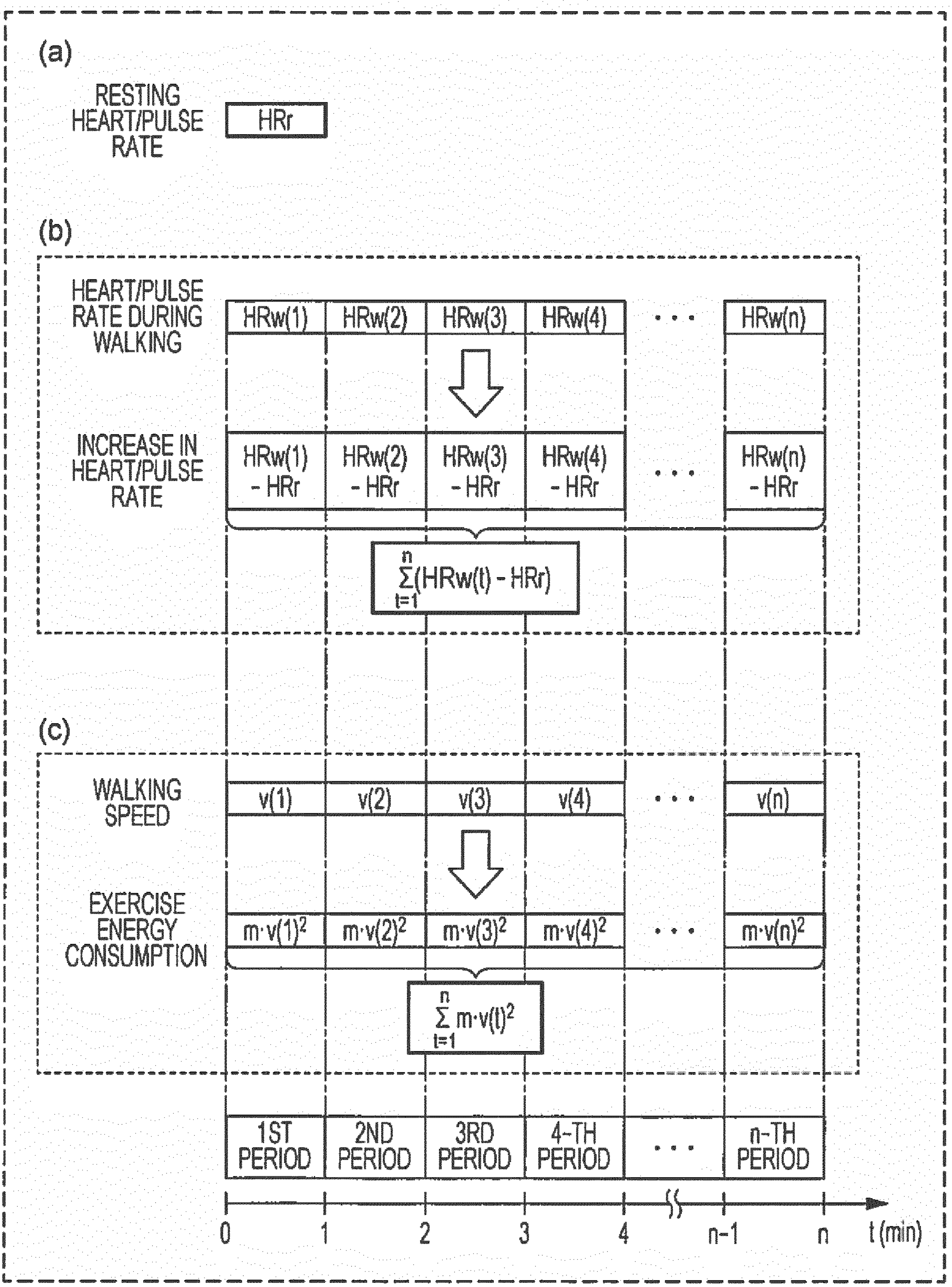
(a)
RESTING HEART/PULSE RATE
HRr
(b)
HEART/PULSE RATE DURING WALKING
HRw(1)
HRw(2)
HRw(3)
HRw(4)
• • •
HRw(n)
INCREASE IN HEART/PULSE RATE
HRw(1) - HRr
HRw(2) - HRr
HRw(3) - HRr
HRw(4) - HRr
• • •
HRw(n) - HRr
$\sum_{t=1}^{n}(HRw(t) - HRr)$
(c)
WALKING SPEED
v(1)
v(2)
v(3)
v(4)
• • •
v(n)
EXERCISE ENERGY CONSUMPTION
$m \cdot v(1)^2$
$m \cdot v(2)^2$
$m \cdot v(3)^2$
$m \cdot v(4)^2$
• • •
$m \cdot v(n)^2$
$\sum_{t=1}^{n} m \cdot v(t)^2$
1ST PERIOD
2ND PERIOD
3RD PERIOD
4-TH PERIOD
• • •
n-TH PERIOD
0
1
2
3
4
n-1
n
t (min)

FIG. 3

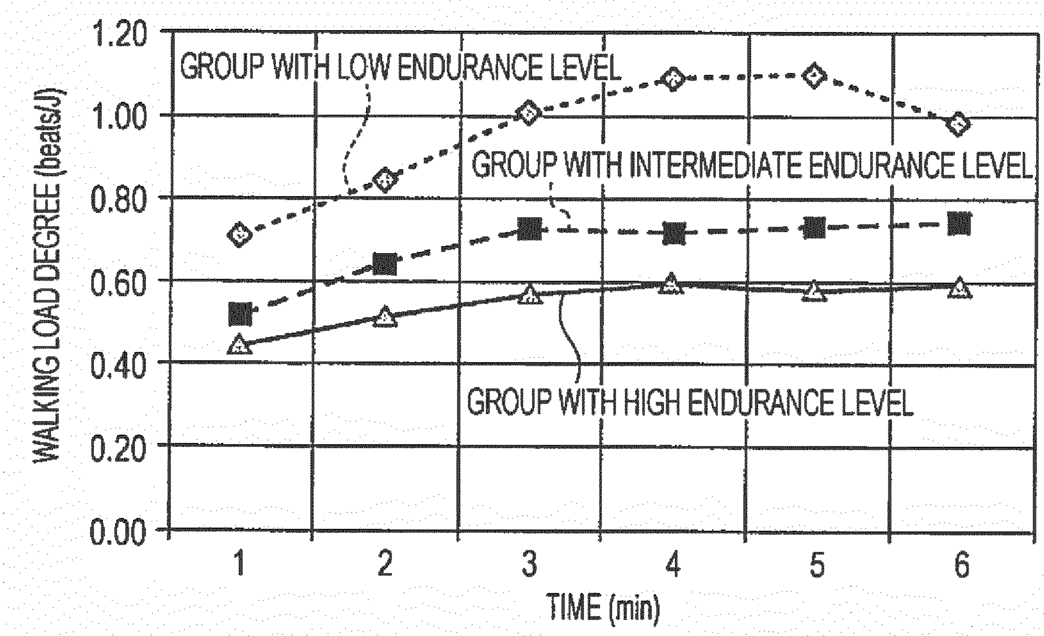
GROUP WITH LOW ENDURANCE LEVEL
GROUP WITH INTERMEDIATE ENDURANCE LEVEL
GROUP WITH HIGH ENDURANCE LEVEL
WALKING LOAD DEGREE (beats/J)
1.20
1.00
0.80
0.60
0.40
0.20
0.00
1
2
3
4
5
6
TIME (min)

FIG. 4

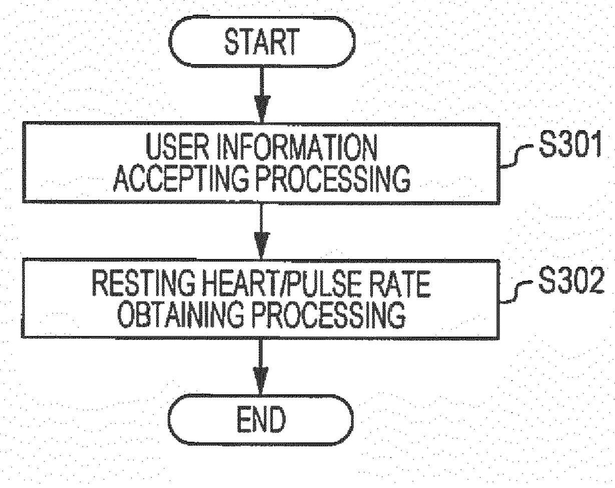
START
USER INFORMATION ACCEPTING PROCESSING
S301
RESTING HEART/PULSE RATE OBTAINING PROCESSING
S302
END

FIG. 5

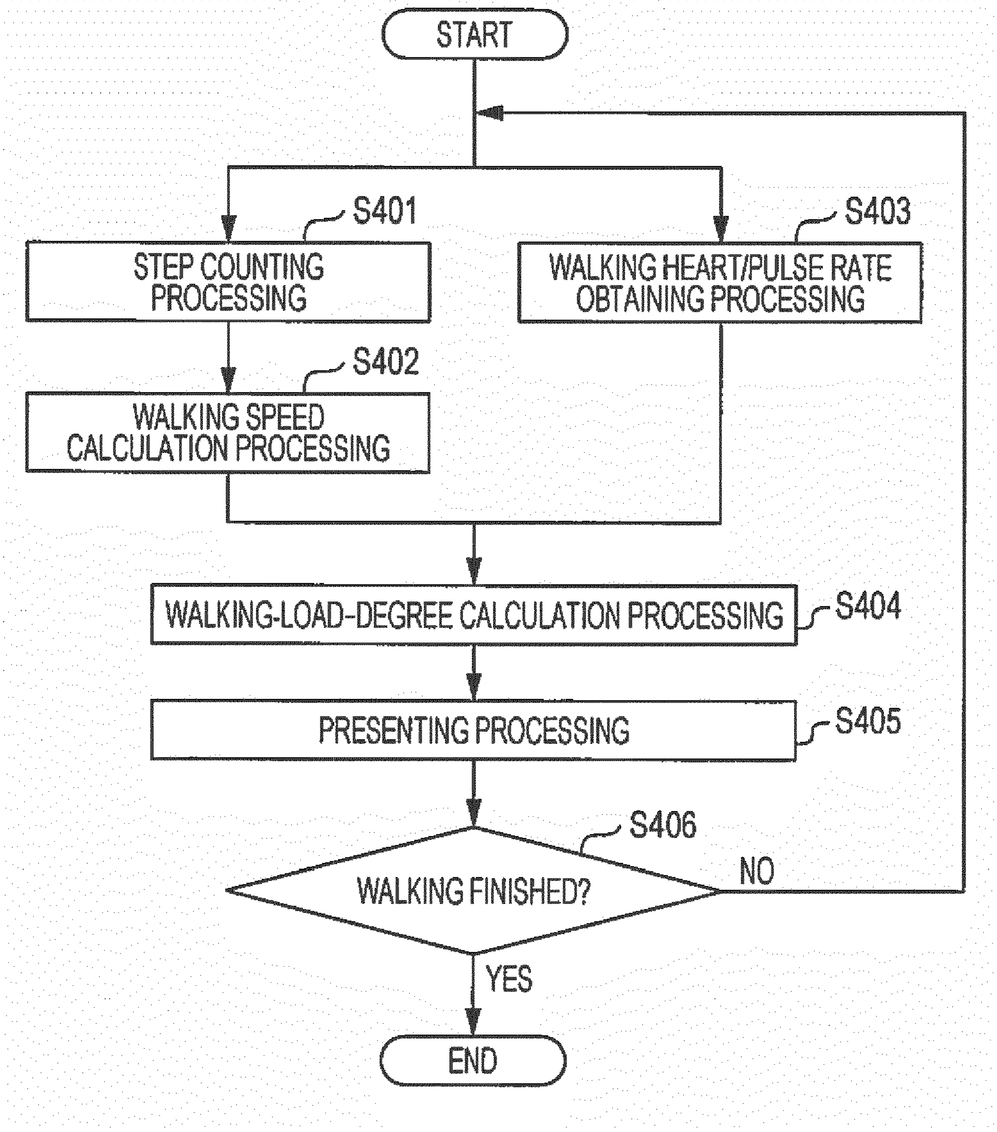
START
S401
STEP COUNTING PROCESSING
S403
WALKING HEART/PULSE RATE OBTAINING PROCESSING
S402
WALKING SPEED CALCULATION PROCESSING
WALKING-LOAD-DEGREE CALCULATION PROCESSING
S404
PRESENTING PROCESSING
S405
S406
WALKING FINISHED?
NO
YES
END

FIG. 6

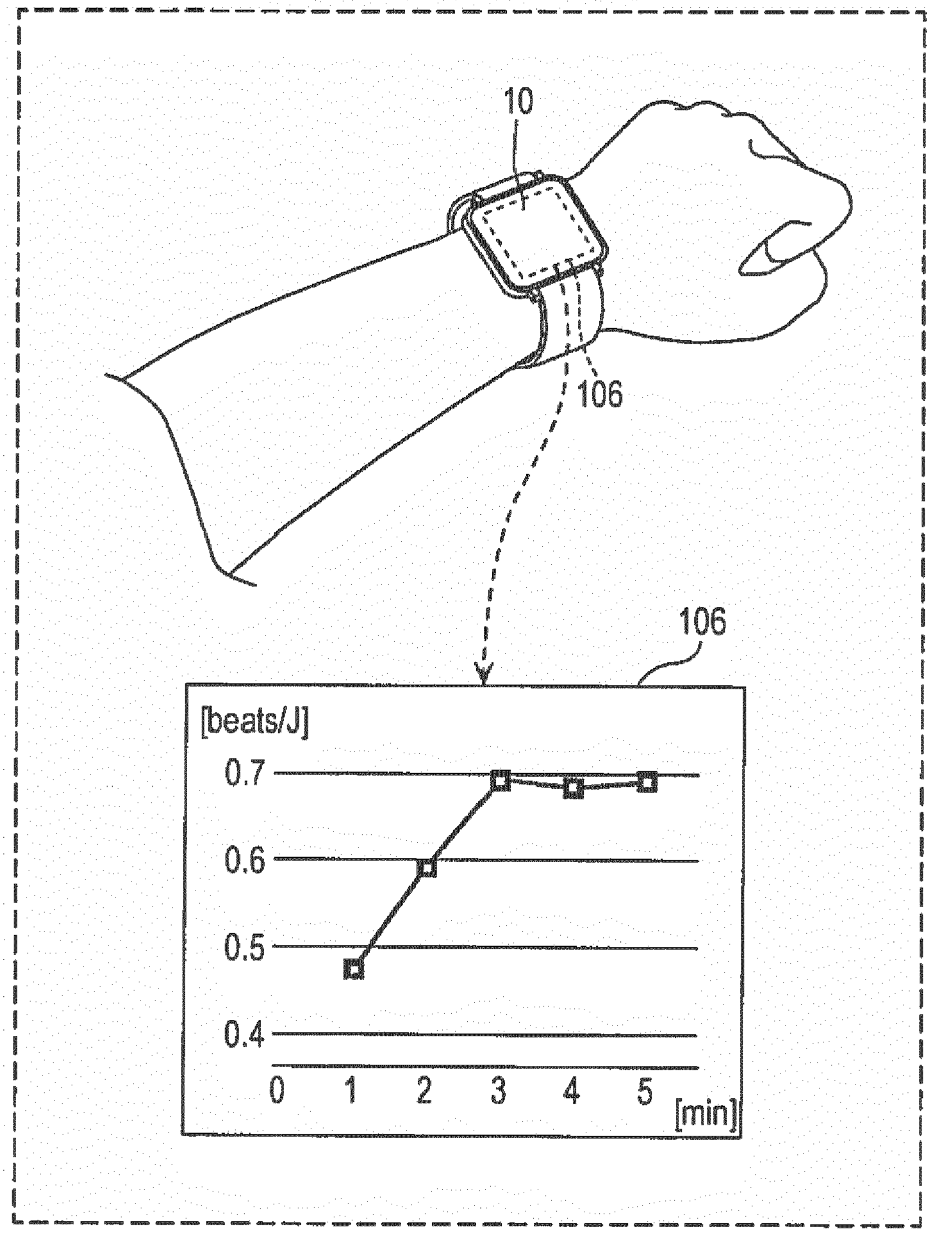

FIG. 7

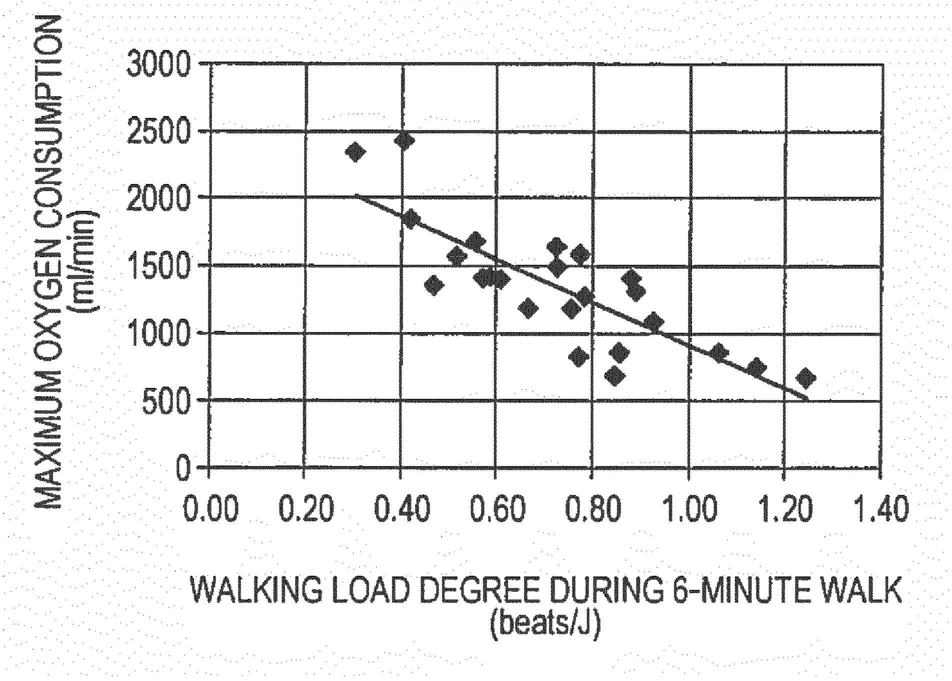
MAXIMUM OXYGEN CONSUMPTION (ml/min)
3000
2500
2000
1500
1000
500
0
0.00 0.20 0.40 0.60 0.80 1.00 1.20 1.40
WALKING LOAD DEGREE DURING 6-MINUTE WALK (beats/J)

FIG. 8

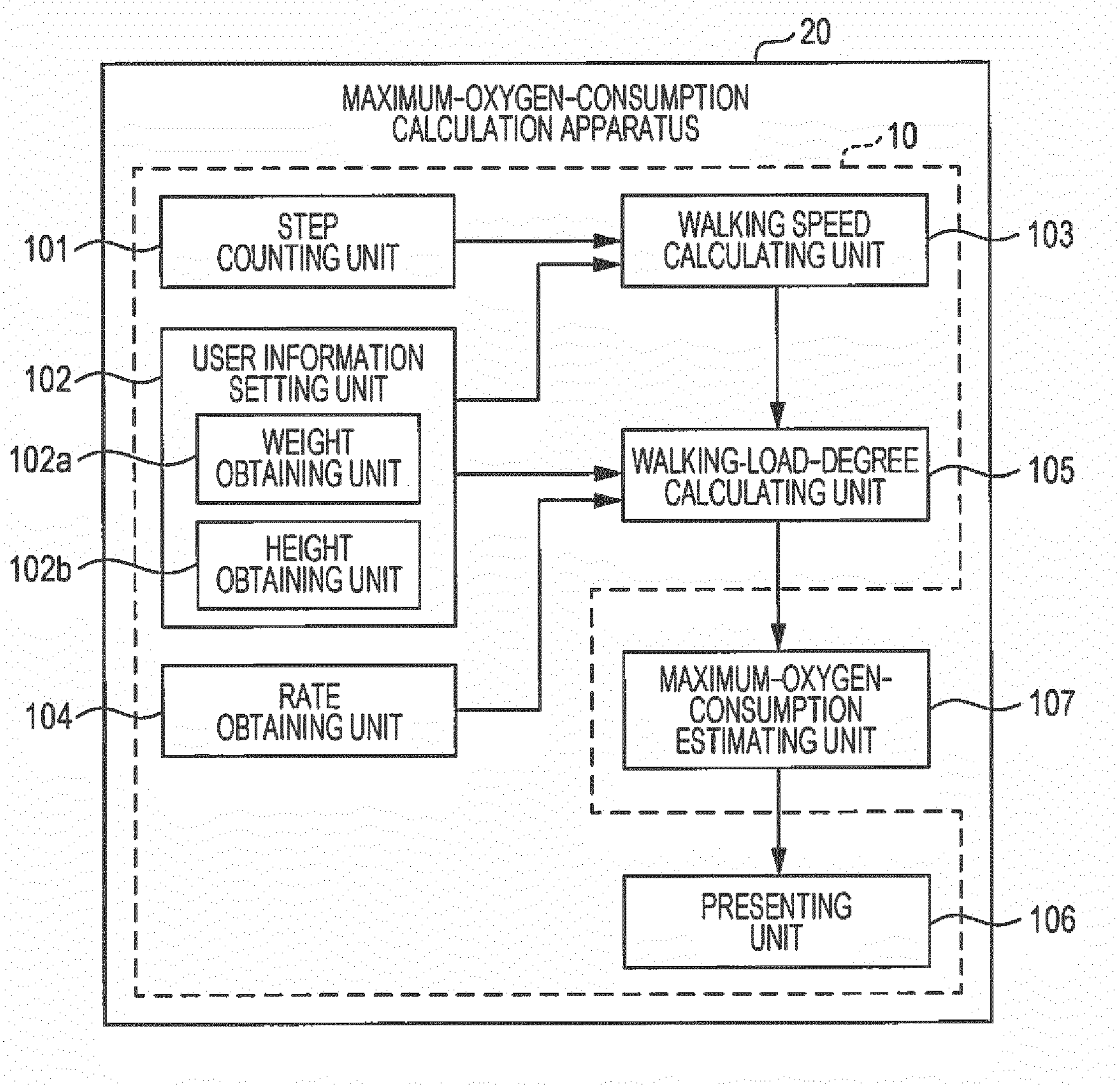
20
MAXIMUM-OXYGEN-CONSUMPTION CALCULATION APPARATUS
10
101
STEP COUNTING UNIT
WALKING SPEED CALCULATING UNIT
103
102
USER INFORMATION SETTING UNIT
102a
WEIGHT OBTAINING UNIT
WALKING-LOAD-DEGREE CALCULATING UNIT
105
102b
HEIGHT OBTAINING UNIT
104
RATE OBTAINING UNIT
MAXIMUM-OXYGEN-CONSUMPTION ESTIMATING UNIT
107
PRESENTING UNIT
106

FIG. 9

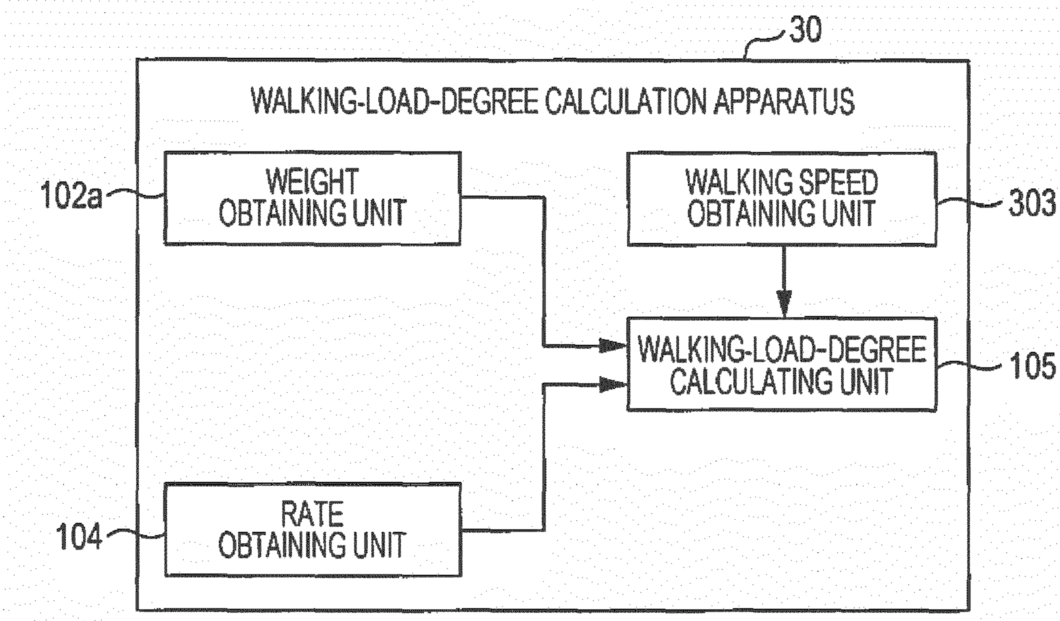
30
WALKING-LOAD-DEGREE CALCULATION APPARATUS
102a
WEIGHT OBTAINING UNIT
WALKING SPEED OBTAINING UNIT
303
WALKING-LOAD-DEGREE CALCULATING UNIT
105
104
RATE OBTAINING UNIT

FIG. 10

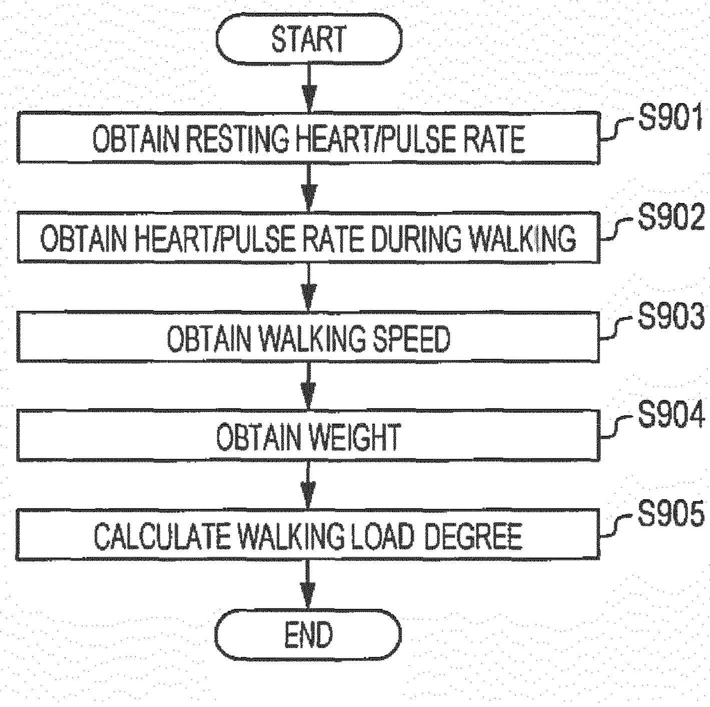
START
OBTAIN RESTING HEART/PULSE RATE
S901
OBTAIN HEART/PULSE RATE DURING WALKING
S902
OBTAIN WALKING SPEED
S903
OBTAIN WEIGHT
S904
CALCULATE WALKING LOAD DEGREE
S905
END

Europäisches Patentamt
European Patent Office
Office européen des brevets

# EUROPEAN SEARCH REPORT

Application Number
EP 16 18 5092

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 1 338 241 A1 (SEIKO EPSON CORP [JP]) 27 August 2003 (2003-08-27)<br>* abstract; claims 1-6; figures 1-27, 49-53 *<br>* paragraph [0007] - paragraph [0012] *<br>* paragraph [0016] - paragraph [0024] *<br>* paragraph [0030] - paragraph [0043] *<br>* paragraph [0085] - paragraph [0137] *<br>----- | 1-8 | INV.<br>G06F19/00 |
| X | KR 2010 0062735 A (KOREA IND TECH INST [KR]) 10 June 2010 (2010-06-10)<br>* abstract; claims 1-10; figures 2,3 *<br>* paragraph [0002] - paragraph [0016] *<br>* paragraph [0022] - paragraph [0040] *<br>----- | 1-8 | |
| X | JP 2013 022256 A (SEIKO EPSON CORP) 4 February 2013 (2013-02-04)<br>* abstract; claims 1-8; figures 1-12 *<br>* paragraph [0009] - paragraph [0023] *<br>* paragraph [0035] - paragraph [0083] *<br>* paragraph [0122] - paragraph [0125] *<br>* paragraph [0098] - paragraph [0110] *<br>* paragraph [0129] - paragraph [0151] *<br>----- | 1-8 | TECHNICAL FIELDS SEARCHED (IPC)<br>G06F |
| X | US 4 566 461 A (LUBELL MICHAEL [US] ET AL) 28 January 1986 (1986-01-28)<br>* abstract; claims 1-17 *<br>* column 2, line 53 - column 4, line 68 *<br>* column 6, line 13 - line 52 *<br>* column 8, line 23 - column 12, line 26 *<br>----- | 1-8 | |

The present search report has been drawn up for all claims

2

| Place of search | Date of completion of the search | Examiner |
|---|---|---|
| Munich | 17 January 2017 | Rákossy, Zoltán |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 16 18 5092

This annex lists the patent family members relating to the patent documents cited in the above-mentioned European search report. The members are as contained in the European Patent Office EDP file on
The European Patent Office is in no way liable for these particulars which are merely given for the purpose of information.

17-01-2017

| Patent document cited in search report | Publication date | Patent family member(s) | Publication date |
|---|---|---|---|
| EP 1338241 A1 | 27-08-2003 | CN 1194574 A | 30-09-1998 |
| | | CN 1545979 A | 17-11-2004 |
| | | CN 1589734 A | 09-03-2005 |
| | | DE 69725095 D1 | 30-10-2003 |
| | | DE 69725095 T2 | 01-04-2004 |
| | | EP 0842635 A1 | 20-05-1998 |
| | | EP 1338241 A1 | 27-08-2003 |
| | | JP 3608204 B2 | 05-01-2005 |
| | | US 6241684 B1 | 05-06-2001 |
| | | WO 9737588 A1 | 16-10-1997 |
| KR 20100062735 A | 10-06-2010 | NONE | |
| JP 2013022256 A | 04-02-2013 | JP 5708341 B2 | 30-04-2015 |
| | | JP 2013022256 A | 04-02-2013 |
| US 4566461 A | 28-01-1986 | EP 0117330 A2 | 05-09-1984 |
| | | JP S59149128 A | 27-08-1984 |
| | | US 4566461 A | 28-01-1986 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 7213499 A
- JP 3608204 B


### Non-patent literature cited in the description


- **ASTRAND PO ; RYHMING I.** A nomogram for calculation of aerobic capacity from pulse rate during submaximal work. *J Appl Physiol,* 1954, vol. 7, 218-221
- **CAHALIN LP et al.** The six-minute walk test predicts peak oxygen uptake and survival in patients with advanced heart failure. *Chest.,* 1996, vol. 110, 325-332